# EUROPEAN PATENT APPLICATION

(11) **EP 4 563 093 A1**
(43) Date of publication of application: **04.06.2025**
(21) Application number: 22953153.8
(22) Date of filing: 28.07.2022
(51) Int. Cl.: A61B 10/00

(54) **ESTIMATION PROGRAM, ESTIMATION METHOD, AND ESTIMATION DEVICE**

(71) Applicant: FUJITSU LIMITED, Kawasaki-shi, Kanagawa 211-8588 (JP)
(72) Inventor: YOUOKU, Sachihiro, Kawasaki-shi, Kanagawa 211-8588 (JP); MI, Xiaoyu, Kawasaki-shi, Kanagawa 211-8588 (JP)
(74) Representative: Hoffmann Eitle
(86) International application number: PCT/JP2022/029204
(87) International publication number: WO 2024/024064

(57) **Abstract**

An estimation device obtains video data including a face of a patient performing a specific task. The estimation device inputs the obtained video data to a first machine learning model to detect occurrence intensity of each of individual action units included in the face of the patient. The estimation device inputs a temporal change in each of the detected occurrence intensity of the plurality of action units to a second machine learning model to estimate a test score of a test tool that executes a test related to dementia.

## Description

### TECHNICAL FIELD

The present invention relates to an estimation program, an estimation method, and an estimation device.

### BACKGROUND ART

It has been conventionally known that a specialty doctor performs a test tool on a subject to diagnose, from a result thereof, dementia in which no basic activities may be performed such as eating, bathing, and the like, and mild cognitive impairment in which no complex activities may be performed such as shopping, housework, and the like while the basic activities may be performed.

### CITATION LIST

### PATENT DOCUMENT

Patent Document 1: Japanese Laid-open Patent Publication No. 2022-61587

### SUMMARY OF INVENTION

### TECHNICAL PROBLEM

However, the test needs to be performed by an examiner with expertise, and the test tool needs a time of 10 to 20 minutes, whereby a test time needed to perform the test tool, obtain the test score, and perform diagnosis is long.

In one aspect, an object is to provide an estimation program, an estimation method, and an estimation device capable of shortening a time for examining a symptom related to dementia.

### SOLUTION TO PROBLEM

According to a first proposal, an estimation program causes a computer to execute a process including: obtaining video data that includes a face of a patient who performs a specific task; detecting occurrence intensity of each of individual action units included in the face of the patient by inputting the obtained video data to a first machine learning model; and estimating a test score of a test tool that executes a test related to dementia by inputting a temporal change in each of the detected occurrence intensity of the plurality of action units to a second machine learning model.

### ADVANTAGEOUS EFFECTS OF INVENTION

According to an embodiment, a time for examining a symptom related to dementia may be shortened.

### BRIEF DESCRIPTION OF DRAWINGS

FIG. 1 is a diagram for explaining an estimation device according to a first embodiment.
FIG. 2 is a functional block diagram illustrating a functional configuration of the estimation device according to the first embodiment.
FIG. 3 is a diagram for explaining exemplary generation of a first machine learning model.
FIG. 4 is a diagram illustrating exemplary arrangement of cameras.
FIGs. 5A-5C are diagrams for explaining movements of markers.
FIG. 6 is a diagram for explaining training of a second machine learning model.
FIG. 7 is a diagram for explaining the MMSE.
FIG. 8 is a diagram for explaining the HDS-R.
FIG. 9 is a diagram for explaining the MoCA.
FIGs. 10A-10C are diagrams illustrating examples of a specific task.
FIG. 11 is a diagram for explaining generation of training data of the second machine learning model.
FIG. 12 is a diagram for explaining estimation of a test score.
FIG. 13 is a diagram for explaining details of the estimation of the test score.
FIG. 14 is a flowchart illustrating a flow of preprocessing.
FIG. 15 is a flowchart illustrating a flow of an estimation process.
FIG. 16 is a diagram for explaining another example of the training data of the second machine learning model.
FIG. 17 is a diagram for explaining an exemplary usage pattern of a test score estimation application.
FIG. 18 is a diagram for explaining an exemplary hardware configuration.

### DESCRIPTION OF EMBODIMENTS

Hereinafter, embodiments of an estimation program, an estimation method, and an estimation device according to the present invention will be described in detail with reference to the drawings. Note that the present invention is not limited by the embodiments. In addition, the individual embodiments may be appropriately combined within a range without inconsistency.

### [First Embodiment]

### <Overall Configuration>

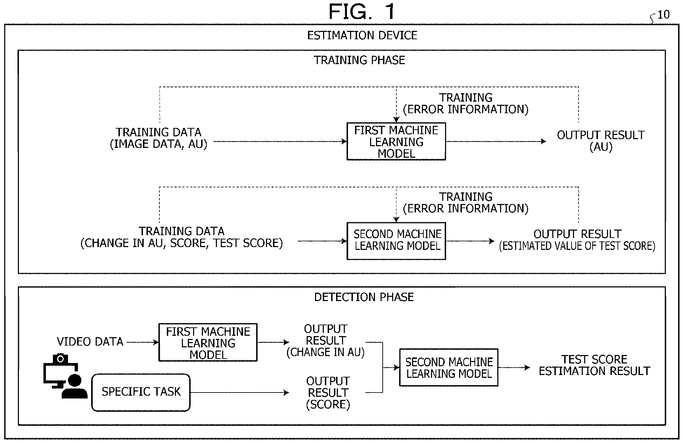

FIG. 1 is a diagram for explaining an estimation device 10 according to a first embodiment. The estimation device 10 illustrated in FIG. 1 is an exemplary computer that estimates a test score of a test tool used by a doctor for diagnosis of dementia from a simple task and facial expression using a technique of facial expression recognition.

Specifically, the estimation device 10 obtains video data including a face of a patient performing a specific task. The estimation device 10 inputs the video data to a first machine learning model, thereby detecting occurrence intensity of each of individual action units (AUs) included in the face of the patient. Thereafter, the estimation device 10 inputs, to a second machine learning model, features including temporal changes in individual pieces of the detected occurrence intensity of the plurality of AUs, thereby estimating the test score of the test tool that executes a test related to dementia.

For example, as illustrated in FIG. 1, in a training phase, the estimation device 10 generates the first machine learning model that outputs the intensity of each AU from image data, and the second machine learning model that outputs the test score from the temporal change in the AU and the score of the specific task.

More specifically, the estimation device 10 inputs, to the first machine learning model, training data having image data in which the face of the patient is captured as an explanatory variable and the occurrence intensity (value) of each AU as an objective variable, and trains parameters of the first machine learning model such that error information between an output result of the first machine learning model and the objective variable is minimized, thereby generating the first machine learning model.

Furthermore, the estimation device 10 inputs, to the second machine learning model, training data having explanatory variables including the temporal change in the occurrence intensity of each AU when the patient is performing the specific task and the score as the execution result of the specific task and the test score as an objective variable, and trains parameters of the second machine learning model such that error information between an output result of the second machine learning model and the objective variable is minimized, thereby generating the second machine learning model.

Thereafter, in a detection phase, the estimation device 10 estimates the test score using the video data when the patient performs the specific task and each of the trained machine learning models.

For example, as illustrated in FIG. 1, the estimation device 10 obtains the video data of the patient who performs the specific task, inputs each frame (image data) in the video data to the first machine learning model as a feature, and obtains the occurrence intensity of each AU for each frame. In this manner, the estimation device 10 obtains a change (change pattern) in the occurrence intensity of each AU of the patient who performs the specific task. Furthermore, the estimation device 10 obtains a score of the specific task after the specific task is complete. Thereafter, the estimation device 10 inputs, to the second machine learning model, the temporal change in the occurrence intensity of each AU of the patient and the score as features, and obtains the test score.

In this manner, with the AUs utilized, the estimation device 10 is enabled to capture a minute change in facial expression with a smaller individual difference, and to estimate the test score of the test tool in a shorter time, whereby a time for examining a symptom related to dementia may be shortened.

### <Functional Configuration>

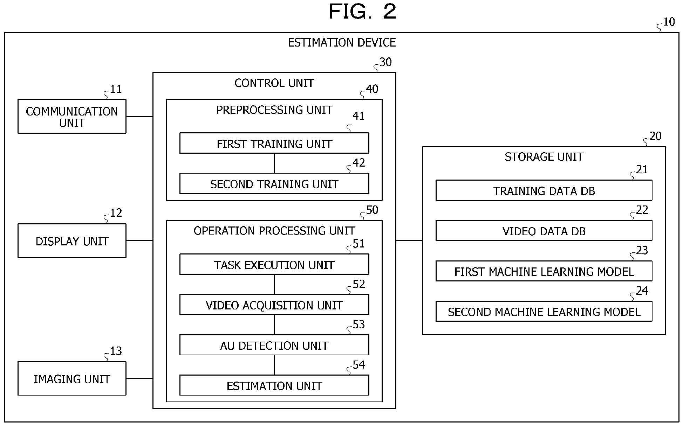

FIG. 2 is a functional block diagram illustrating a functional configuration of the estimation device 10 according to the first embodiment. As illustrated in FIG. 2, the estimation device 10 includes a communication unit 11, a display unit 12, an imaging unit 13, a storage unit 20, and a control unit 30.

The communication unit 11 is a processing unit that controls communication with another device, and is implemented by, for example, a communication interface or the like. For example, the communication unit 11 receives video data and a score of a specific task to be described later, and transmits, using the control unit 30 to be described later, a processing result to a destination specified in advance.

The display unit 12 is a processing unit that displays and outputs various types of information, and is implemented by, for example, a display, a touch panel, or the like. For example, the display unit 12 outputs a specific task, and receives a response to the specific task.

The imaging unit 13 is a processing unit that captures video to obtain video data, and is implemented by, for example, a camera or the like. For example, the imaging unit 13 captures video including the face of the patient while the patient is performing a specific task, and stores it in the storage unit 20 as video data.

The storage unit 20 is a processing unit that stores various types of data, programs to be executed by the control unit 30, and the like, and is implemented by, for example, a memory, a hard disk, or the like. The storage unit 20 stores a training data database (DB) 21, a video data DB 22, a first machine learning model 23, and a second machine learning model 24.

The training data DB 21 is a database for storing various types of training data to be used to generate the first machine learning model 23 and the second machine learning model 24. The training data stored here may include supervised training data to which ground truth information is attached, and unsupervised training data to which no ground truth information is attached.

The video data DB 22 is a database that stores video data captured by the imaging unit 13. For example, the video data DB 22 stores, for each patient, video data including the face of the patient while performing a specific task. Note that the video data includes a plurality of time-series frames. A frame number is assigned to each of the frames in time-series ascending order. One frame is image data of a still image captured by the imaging unit 13 at certain timing.

The first machine learning model 23 is a machine learning model that outputs occurrence intensity of each AU in response to an input of each frame (image data) included in the video data. Specifically, the first machine learning model 23 estimates a certain AU by a technique of separating and quantifying a facial expression based on facial parts and facial expression muscles. The first machine learning model 23 outputs, in response to the input of the image data, a facial expression recognition result such as "AU 1: 2, AU 2: 5, AU 3: 1, ..." expressing the occurrence intensity (e.g., on a five-point scale) of each of AUs from an AU 1 to an AU 28 set to specify the facial expression. For example, various algorithms such as a neural network and a random forest may be adopted as the first machine learning model 23.

The second machine learning model 24 is a machine learning model that outputs an estimation result of a test score in response to an input of a feature. For example, the second machine learning model 24 outputs the estimation result including the test score in response to the input of the features including a temporal change (change pattern) of the occurrence intensity of each AU and the score of the specific task. For example, various algorithms such as a neural network and a random forest may be adopted as the second machine learning model 24.

The control unit 30 is a processing unit that takes overall control of the estimation device 10, and is implemented by, for example, a processor or the like. The control unit 30 includes a preprocessing unit 40 and an operation processing unit 50. Note that the preprocessing unit 40 and the operation processing unit 50 are implemented by an electronic circuit included in a processor, a process executed by the processor, or the like.

### (Preprocessing Unit 40)

The preprocessing unit 40 is a processing unit that executes generation of each model using the training data stored in the storage unit 20 prior to the operation of the test score estimation. The preprocessing unit 40 includes a first training unit 41 and a second training unit 42.

The first training unit 41 is a processing unit that executes generation of the first machine learning model 23 through training using training data. Specifically, the first training unit 41 generates the first machine learning model 23 through supervised training using training data to which ground truth information (label) is attached.

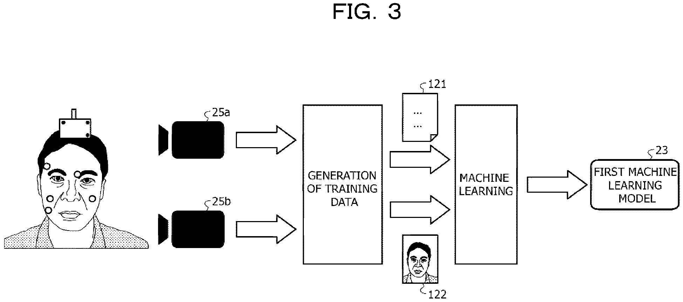

Here, the generation of the first machine learning model 23 will be described with reference to FIGs. 3 to 5A-5C. FIG. 3 is a diagram for explaining exemplary generation of the first machine learning model 23. As illustrated in FIG. 3, the first training unit 41 generates training data and performs machine learning on image data captured by each of a red-green-blue (RGB) camera 25a and an infrared (IR) camera 25b.

As illustrated in FIG. 3, first, the RGB camera 25a and the IR camera 25b are directed to a face of a person to which markers are attached. For example, the RGB camera 25a is a common digital camera, which receives visible light to generate an image. Furthermore, for example, the IR camera 25b senses infrared rays. Furthermore, the markers are, for example, IR reflection (retroreflection) markers. The IR camera 25b is capable of performing motion capture by using the IR reflection by the markers. Furthermore, in the following descriptions, a person to be captured will be referred to as a subject.

In the process of generating the training data, the first training unit 41 obtains the image data captured by the RGB camera 25a, and a result of the motion capture by the IR camera 25b. Then, the first training unit 41 generates occurrence intensity 121 of an AU and image data 122 obtained by deleting the markers from the captured image data through image processing. For example, the occurrence intensity 121 may be data in which the occurrence intensity of each AU is expressed on a five-point scale of A to E and annotated as "AU 1: 2, AU 2: 5, AU 3: 1, ...".

In the machine learning process, the first training unit 41 carries out the machine learning using the occurrence intensity 121 of the AUs and the image data 122 output from the process of generating the training data, and generates the first machine learning model 23 for estimating occurrence intensity of an AU from image data. The first training unit 41 may use the occurrence intensity of an AU as a label.

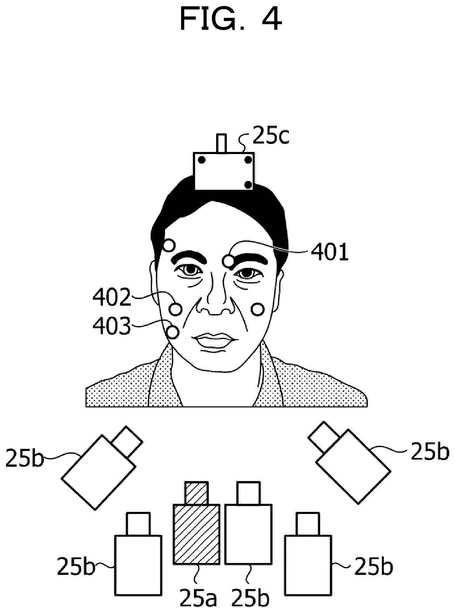

Here, arrangement of cameras will be described with reference to FIG. 4. FIG. 4 is a diagram illustrating exemplary arrangement of cameras. As illustrated in FIG. 4, a plurality of the IR cameras 25b may form a marker tracking system. In that case, the marker tracking system may detect positions of IR reflection markers by stereo imaging. Furthermore, it is assumed that a relative positional relationship between each of the plurality of IR cameras 25b is corrected in advance by camera calibration.

Furthermore, a plurality of markers is attached to the face of the subject to be imaged to cover the AU 1 to the AU 28. Positions of the markers change according to a change in facial expression of the subject. For example, a marker 401 is arranged near the root of the eyebrow. In addition, a marker 402 and a marker 403 are arranged near the nasolabial line. The markers may be arranged over the skin corresponding to movements of one or more AUs and facial expression muscles. Furthermore, the markers may be arranged to exclude a position above the skin where a texture change is larger due to wrinkles or the like.

Moreover, the subject wears an instrument 25c to which a reference point marker is attached outside the contour of the face. It is assumed that a position of the reference point marker attached to the instrument 25c does not change even when the facial expression of the subject changes. Accordingly, the first training unit 41 is enabled to detect a positional change of the markers attached to the face based on a change in the position relative to the reference point marker. Furthermore, with the number of the reference point markers set to three or more, the first training unit 41 is enabled to specify the position of the marker in the three-dimensional space.

The instrument 25c is, for example, a headband. In addition, the instrument 25c may be a virtual reality (VR) headset, a mask made of a hard material, or the like. In that case, the first training unit 41 may use a rigid surface of the instrument 25c as a reference point marker.

Note that, when the IR camera 25b and the RGB camera 25a capture images, the subject changes facial expressions. Accordingly, a manner of time-series changing of the facial expressions may be obtained as images. In addition, the RGB camera 25a may capture a moving image. A moving image may be regarded as a plurality of still images arranged in time series. Furthermore, the subject may change the facial expression freely, or may change the facial expression according to a predefined scenario.

Note that the occurrence intensity of an AU may be determined based on a movement amount of a marker. Specifically, the first training unit 41 may determine the occurrence intensity based on the movement amount of the marker calculated based on a distance between a position preset as a determination criterion and the position of the marker.

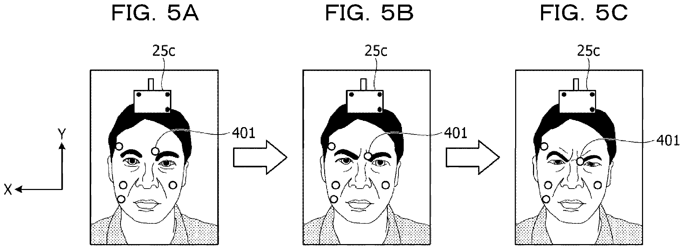

Here, movements of markers will be described with reference to FIGS. 5A-5C. FIGS. 5A-5C are diagrams for explaining movements of markers. In FIGs. 5A-5C are images captured by the RGB camera 25a. In addition, the images are assumed to be captured in the order of FIG. 5A, FIG. 5B, and FIG. 5C. For example, (a) is an image when the subject is expressionless. The first training unit 41 may regard the positions of the markers in the image (a) as reference positions at which the movement amount is zero. As illustrated in FIG. 5, the subject has a facial expression of drawing the eyebrows together. At this time, the position of the marker 401 moves downward as the facial expression changes. At this time, the distance between the position of the marker 401 and the reference point marker attached to the instrument 25c increases.

In this manner, the first training unit 41 specifies the image data in which a certain facial expression of the subject is captured and the intensity of each marker at the time of the facial expression, and generates training data having an explanatory variable "image data" and an objective variable "intensity of each marker". Then, the first training unit 41 carries out supervised training using the generated training data to generate the first machine learning model 23. For example, the first machine learning model 23 is a neural network. The first training unit 41 carries out the machine learning of the first machine learning model 23 to change parameters of the neural network. The first training unit 41 inputs the explanatory variable to the neural network. Then, the first training unit 41 generates a machine learning model in which the parameters of the neural network are changed to reduce an error between an output result output from the neural network and ground truth data, which is the objective variable.

Note that the generation of the first machine learning model 23 is merely an example, and other approaches may be used. Furthermore, a model disclosed in Japanese Laid-open Patent Publication No. 2021-111114 may be used as the first machine learning model 23. Furthermore, face orientation may also be trained through a similar approach.

The second training unit 42 is a processing unit that executes generation of the second machine learning model 24 through training using training data. Specifically, the second training unit 42 generates the second machine learning model 24 through supervised training using training data to which ground truth information (label) is attached.

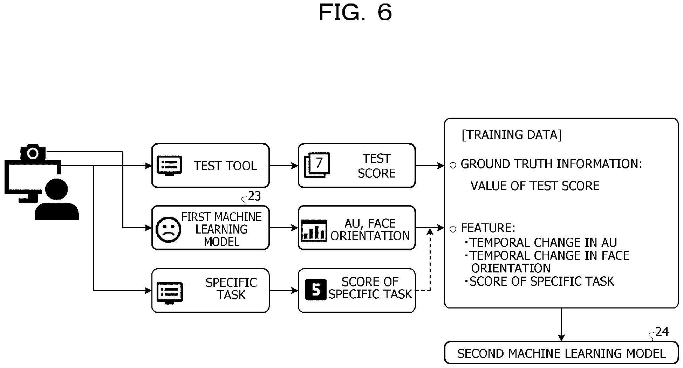

FIG. 6 is a diagram for explaining training of the second machine learning model 24. As illustrated in FIG. 6, the second training unit 42 may train the second machine learning model 24 using training data prepared in advance or training data generated using video data when the patient is performing a specific task and the trained first machine learning model 23.

For example, the second training unit 42 obtains the "test score value" of the test tool performed on the patient by the doctor. Furthermore, the second training unit 42 obtains the score, which is a result of the execution of the specific task by the patient, and the occurrence intensity and the face orientation of each AU obtained by inputting the video data including the face of the patient captured while the patient is performing the specific task to the first machine learning model 23.

Then, the second training unit 42 generates training data including the "test score value" as "ground truth information" and the "temporal change in the occurrence intensity of each AU, temporal change in the face orientation, and score of the specific task" as "features". Then, the second training unit 42 inputs the features of the training data to the second machine learning model 24, and updates the parameters of the second machine learning model 24 such that the error between the output result of the second machine learning model 24 and the ground truth information is made smaller.

Here, the test tool will be described. As the test tool, the mini mental state examination (MMSE) or the Hasegawa's dementia scale-revised (HDS-R) used for a test related to dementia, or a test tool for executing a test related to dementia such as the Montreal cognitive assessment (MoCA) may be used.

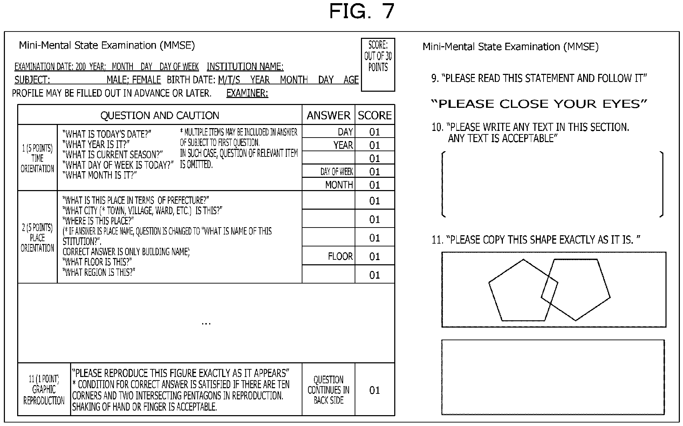

FIG. 7 is a diagram for explaining the MMSE. The MMSE illustrated in FIG. 7 is a cognitive brain test with 11 items and 30 points using verbal, written, and drawn answer methods, and needs a timescale of 6 to 10 minutes. The test is performed such as "time orientation, delayed reproduction of three words, recitation of characters, transcription of characters, place orientation, calculation, three-step verbal instruction, graphic reproduction, immediate reproduction of three words, object designation, transcription instruction", and the like. Scores are determined as determination criteria, and dementia is suspected when the score is 23 points or less, and mild cognitive impairment (MCI) is suspected when the score is 27 points or less. For example, as determination criteria for each score, 0 to 10 points are set as severe, 11 to 20 points are set as moderate, 21 to 27 points are set as mild, and 28 to 30 points are set as no problem.

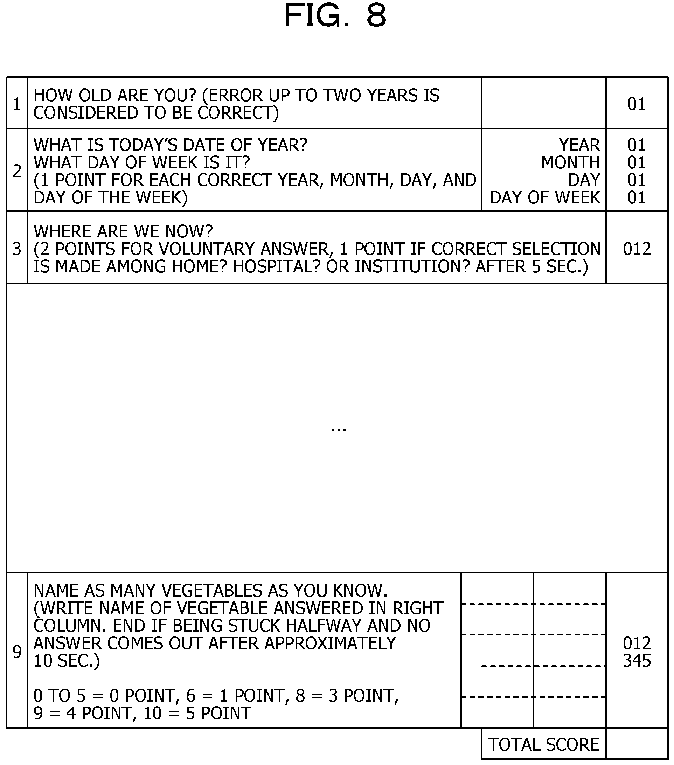

FIG. 8 is a diagram for explaining the HDS-R. The HDS-R illustrated in FIG. 8 is a cognitive brain test with 9 items and 30 points using only a verbal answer method, and needs a timescale of 6 to 10 minutes. The test is performed such as "age, time orientation, place orientation, immediate memorization of three words, delayed reproduction of three words, calculation, reverse numeric reading, item memorization, language fluency", and the like. Scores are determined as determination criteria, and dementia is suspected when the score is 20 points or less. For example, as determination criteria for each severity level, non-dementia is set at around 24.45 points, mild dementia is set at around 17.85 points, moderate dementia is set at around 14.10, slightly severe dementia is set at around 9.23 points, and severe dementia is set at around 4.75 points.

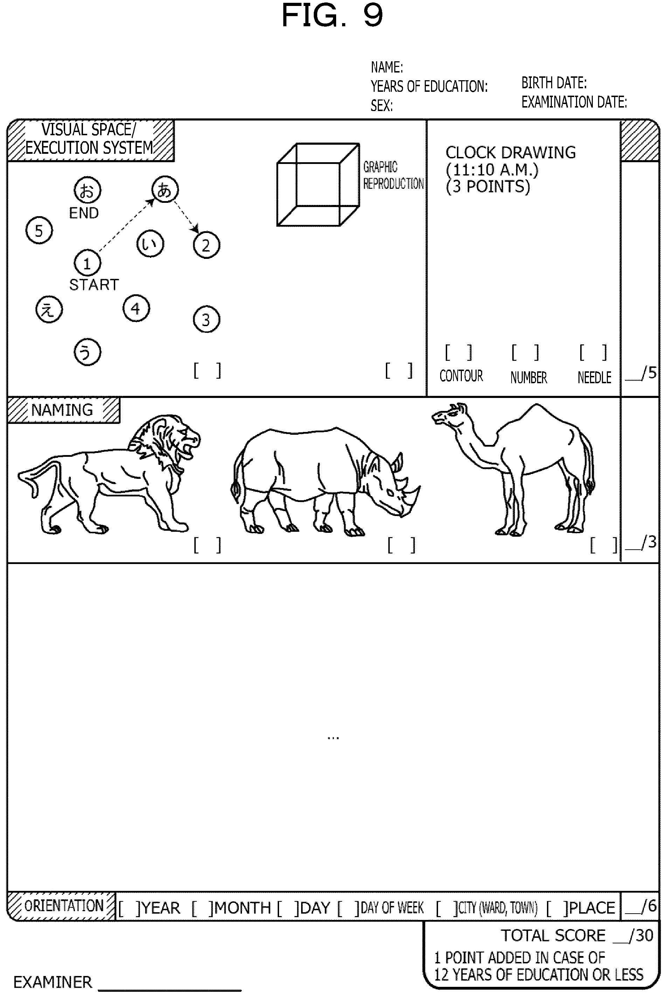

FIG. 9 is a diagram for explaining the MoCA. The MoCA illustrated in FIG. 9 uses verbal, written, and drawn answer methods, and needs a timescale of approximately 10 minutes. As contents of the test, "visuospatial executive function, naming, memory, attention, recitation, word recall, abstract concept, delayed reproduction, orientation", and the like are performed. Scores are determined as determination criteria, and MCI is suspected when the score is 25 points or less. The MoCA is basically for MCI screening.

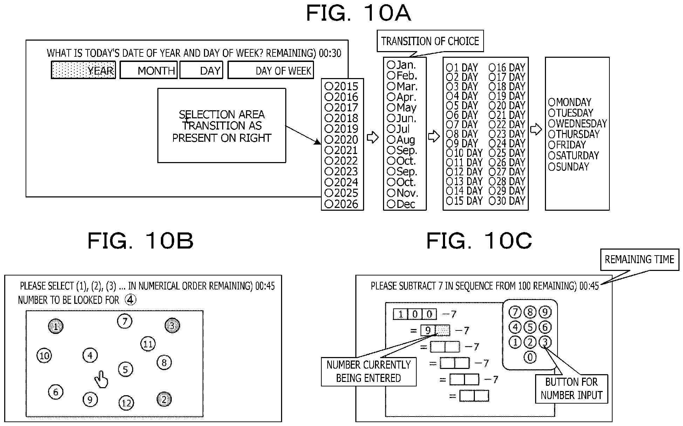

Next, a specific task will be described. FIGs. 10A-10C are diagrams illustrating examples of the specific task. The specific task illustrated in FIGs. 10A-10C are examples of an application or an interactive application that tests a cognitive function by loading the cognitive function. The specific task is a tool that may be readily available to the patient in a shorter time as compared with a rigid test tool used by the doctor.

For example, the specific task illustrated in FIG. 10A is a task for causing the patient to select today's date. The selection is made using radio buttons, and the year, month, day, and day of the week are selected in that order starting with the year. It ends when the answer is complete or the time limit is exceeded. The response completion time and the answer are registered as scores. Note that the halfway answer and the time limit are the scores when time runs out.

The specific task illustrated in FIG. 10B is a task of being caused to select numbers, which are randomly arranged and displayed, in the order starting with "1". Clicking on 1 enables clicking on 2, and clicking on 2 enables clicking on 3. The selected numbers are displayed in a different color, and the number currently being searched for and the remaining time are displayed outside the frame of the task. When the displayed number is XX, XX pieces are complete, but the task ends when the time limit of YY seconds is exceeded. The completion time and the number of achievements (number of correct answers) are registered as scores.

The specific task illustrated in FIG. 10C is a task of being caused to subtract 7 in sequence from displayed 100. The item currently being entered is displayed in a different color, and the task is terminated after the maximum number (XX) of calculations. The task ends when XX calculations are complete or the time limit of YY seconds is exceeded. The completion time and the answer are registered as scores. Note that the halfway answer and the time limit are the scores when time runs out.

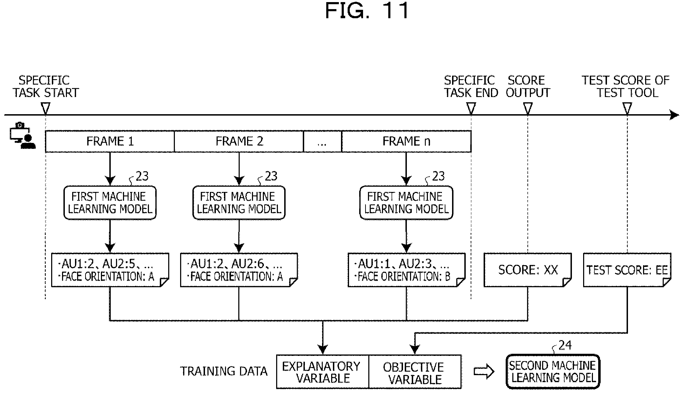

Next, the generation of the training data will be described in detail. FIG. 11 is a diagram for explaining the generation of the training data of the second machine learning model 24. As illustrated in FIG. 11, the second training unit 42 obtains, from a camera or the like, video data captured from the start to the end of the specific task, and obtains "occurrence intensity of each AU" and "face orientation" from each frame of the video data.

For example, the second training unit 42 inputs the image data of the first frame to the trained first machine learning model 23, and obtains "AU 1: 2, AU 2: 5 ..." and "face orientation: A". Likewise, the second training unit 42 inputs the image data of the second frame to the trained first machine learning model 23, and obtains "AU 1: 2, AU 2: 6 ..." and "face orientation: A". In this manner, the second training unit 42 specifies, from the video data, the temporal change in each AU of the patient and the temporal change in the face orientation of the patient.

Furthermore, the second training unit 42 obtains a score "XX" output after the completion of the specific task. Furthermore, the second training unit 42 obtains, from the doctor, an electronic medical chart, or the like, "test score: EE", which is a result (value) of the test tool performed by the doctor on the patient who has performed the specific task.

Then, the second training unit 42 generates training data in which the "occurrence intensity of each AU" and the "face orientation" obtained using each frame and the "score (XX)" are used as explanatory variables and the "test score: EE" is used as an objective variable, and generates the second machine learning model 24. That is, the second machine learning model 24 trains the relationship between the "test score: EE" and the "change pattern of the temporal change in the occurrence intensity of each AU, change pattern of the temporal change in the face orientation, and score".

### (Operation Processing Unit 50)

Returning to FIG. 2, the operation processing unit 50 is a processing unit that includes a task execution unit 51, a video acquisition unit 52, an AU detection unit 53, and an estimation unit 54, and estimates a test score of a person (patient) who appears in the video data using each model prepared in advance by the preprocessing unit 40.

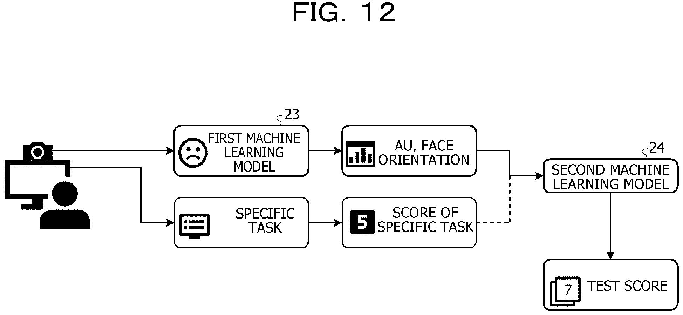

Here, the estimation of the test score will be described with reference to FIG. 12. FIG. 12 is a diagram for explaining the estimation of the test score. As illustrated in FIG. 12, the operation processing unit 50 inputs video data including the face of the patient performing a specific task to the trained first machine learning model 23, and specifies a temporal change in each AU of the patient and a temporal change in the face orientation of the patient. Furthermore, the operation processing unit 50 obtains the score of the specific task. Then, the operation processing unit 50 inputs the temporal change in the AUs, the temporal change in the face orientation, and the score to the second machine learning model 24, and estimates a value of the test score.

The task execution unit 51 is a processing unit that performs a specific task on the patient and obtains a score. For example, the task execution unit 51 displays any of the tasks illustrated in FIGs. 10A-10C on the display unit 12, and receives an answer (input) from the patient, thereby executing the specific task. Thereafter, upon completion of the specific task, the task execution unit 51 obtains a score and outputs it to the estimation unit 54 and the like.

The video acquisition unit 52 is a processing unit that obtains video data including the face of the patient performing a specific task. For example, the video acquisition unit 52 starts imaging using the imaging unit 13 when the specific task starts, ends the imaging using the imaging unit 13 when the specific task ends, and obtains the video data during the execution of the specific task from the imaging unit 13. Then, the video acquisition unit 52 stores the obtained video data in the video data DB 22, and outputs it to the AU detection unit 53.

The AU detection unit 53 is a processing unit that detects occurrence intensity of each AU included in the face of the patient by inputting the video data obtained by the video acquisition unit 52 to the first machine learning model 23. For example, the AU detection unit 53 extracts each frame from the video data, inputs each frame to the first machine learning model 23, and detects the occurrence intensity of the AUs and the face orientation of the patient for each frame. Then, the AU detection unit 53 outputs, to the estimation unit 54, the occurrence intensity of the AUs and the face orientation of the patient for each detected frame. Note that the face orientation may be specified from the occurrence intensity of the AUs.

The estimation unit 54 is a processing unit that estimates a test score, which is a result of execution of the test tool, using the temporal change in the occurrence intensity of each AU, the temporal change in the face orientation of the patient, and the score of the specific task as features. For example, the estimation unit 54 inputs, to the second machine learning model 24, the "score" obtained by the task execution unit 51, the "temporal change in the occurrence intensity of each AU" obtained by linking, in time series, the "occurrence intensity of each AU" detected by the AU detection unit for each frame, and the "temporal change in the face orientation" obtained by linking, in time series, the "face orientation" detected in a similar manner, as features. Then, the estimation unit 54 obtains an output result of the second machine learning model 24, and obtains, as an estimation result of the test score, a value having the largest probability value among the probability values (reliability) of the individual values of the test score included in the output result. Thereafter, the estimation unit 54 displays and outputs the estimation result on the display unit 12, and stores it in the storage unit 20.

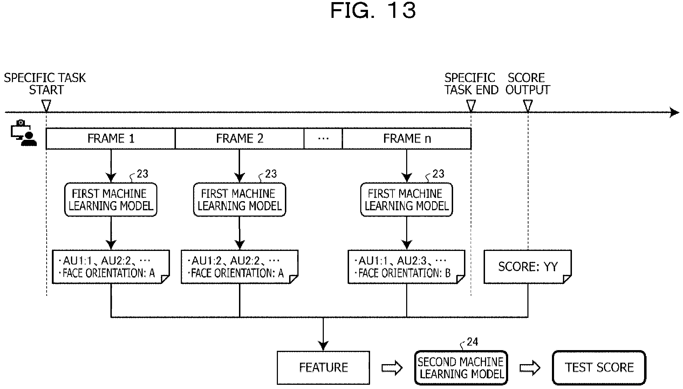

Here, details of the estimation of the test score will be described. FIG. 13 is a diagram for explaining details of the estimation of the test score. As illustrated in FIG. 13, the operation processing unit 50 obtains video data captured from the start to the end of the specific task, and obtains "occurrence intensity of each AU" and "face orientation" from each frame of the video data.

For example, the operation processing unit 50 inputs the image data of the first frame to the trained first machine learning model 23, and obtains "AU 1: 2, AU 2: 5 ..." and "face orientation: A". Likewise, the operation processing unit 50 inputs the image data of the second frame to the trained first machine learning model 23, and obtains "AU 1: 2, AU 2: 5 ..." and "face orientation: A". In this manner, the operation processing unit 50 specifies, from the video data, the temporal change in each AU of the patient and the temporal change in the face orientation of the patient.

Thereafter, the operation processing unit 50 obtains the score "YY" of the specific task, inputs, to the second machine learning model 24, the "temporal change in each AU of the patient (AU 1: 2, AU 2: 5 ..., AU 1: 2, AU 2: 5 ...), temporal change in the face orientation of the patient (face orientation: A, face orientation: A, ...), and score (YY)" as features, and estimates a value of the test score.

### <Flow of Preprocessing>

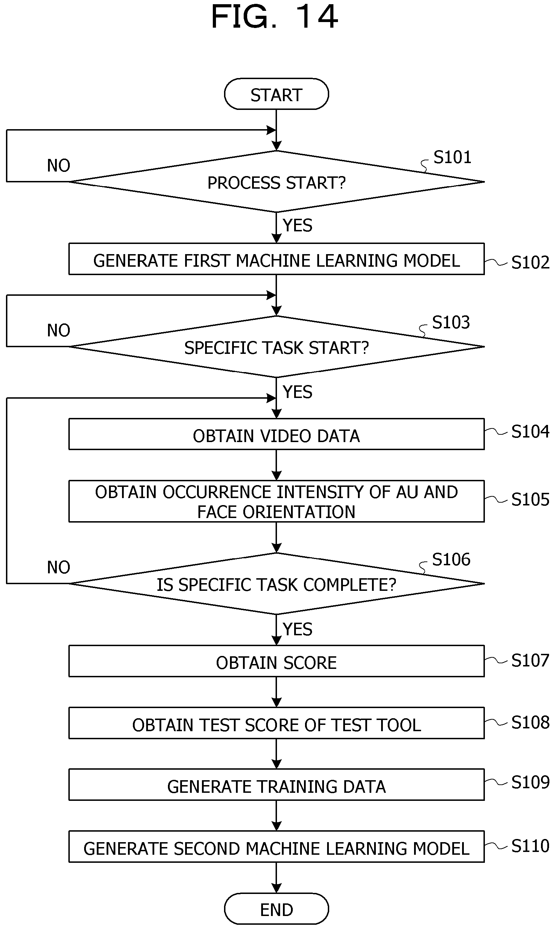

FIG. 14 is a flowchart illustrating a flow of the preprocessing. As illustrated in FIG. 14, when a process start is instructed (Yes in S101), the preprocessing unit 40 generates the first machine learning model 23 using the training data (S102).

Subsequently, when the specific task starts (Yes in S103), the preprocessing unit 40 obtains video data (S104). Then, the preprocessing unit 40 inputs each frame of the video data to the first machine learning model 23, and obtains, for each frame, the occurrence intensity of each AU and the face orientation (S105).

Thereafter, when the specific task is complete (Yes in S106), the preprocessing unit 40 obtains a score (S107). Furthermore, the preprocessing unit 40 obtains an execution result (test score) of the test tool (S108).

Then, the preprocessing unit 40 generates training data including the temporal change in the occurrence intensity of each AU, the temporal change in the face orientation, and the score (S109), and generates the second machine learning model 24 using the training data (S110).

### <Flow of Estimation Process>

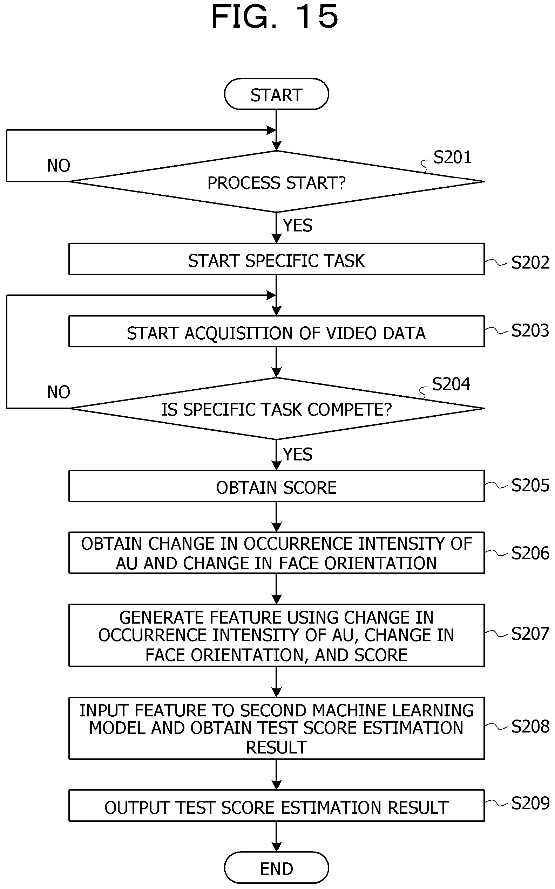

FIG. 15 is a flowchart illustrating a flow of the estimation process. As illustrated in FIG. 15, when a process start is instructed (Yes in S201), the operation processing unit 50 performs the specific task on the patient (S202), and starts acquisition of video data (S203).

Then, when the specific task is complete (Yes in S204), the operation processing unit 50 obtains a score, and ends the acquisition of the video data (S205). Then, the operation processing unit 50 inputs each frame of the video data to the first machine learning model 23, and obtains, for each frame, the occurrence intensity of each AU and the face orientation (S206).

Thereafter, the operation processing unit 50 specifies the temporal change in each AU and the temporal change in the face orientation based on the occurrence intensity of each AU and the face orientation for each frame, and generates the "temporal change in each AU, temporal change in the face orientation, and score" as features (S207).

Then, the operation processing unit 50 inputs the features to the second machine learning model 24, obtains an estimation result by the second machine learning model 24 (S208), and outputs the estimation result to the display unit 12 or the like (S209).

### <Effects>

As described above, the estimation device 10 according to the first embodiment may estimate a test score of the cognitive function to perform screening for dementia and mild cognitive impairment even without the expertise of the doctor. Furthermore, the estimation device 10 according to the first embodiment may screen dementia and mild cognitive impairment in a shorter time by combining a specific task that takes only a few minutes and facial expression information as compared with the case of diagnosis using a test tool.

### [Second Embodiment]

Although the embodiment of the present invention has been described above, the present invention may be implemented in various different modes in addition to the embodiment described above.

### (Training Data)

While the example of using the temporal change in each AU, the temporal change in the face orientation, and the score as the features (explanatory variables) for the training data of the second machine learning model 24 has been described in the first embodiment described above, it is not limited to this.

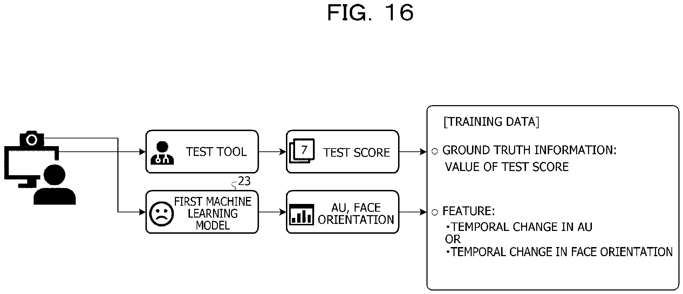

FIG. 16 is a diagram for explaining another example of training data of a second machine learning model 24. As illustrated in FIG. 16, an estimation device 10 may use, for example, only a temporal change in each AU as an explanatory variable, or may use the temporal change in each AU and a temporal change in face orientation as explanatory variables. In addition, although illustration is omitted, the temporal change in each AU and a score may be used as explanatory variables.

Furthermore, while the example of using a value of the test score as an objective variable has been described in the embodiment above, it is not limited to this. For example, a range of a test score may be used as an objective variable, such as "0 to 10 points", "11 to 20 points", or "20 to 30 points".

As described above, since the estimation device 10 may determine a feature to be used for training and detection according to accuracy and cost, a simple service may be provided, and a detailed service for supporting diagnosis of a doctor may also be provided.

### (Rule Base)

While the example of estimating a test score using the second machine learning model 24 has been described in the embodiment above, it is not limited to this. For example, a test score may be estimated using a detection rule in which a combination of a pattern of the temporal change in each AU and a pattern of the temporal change in the face orientation is associated with a test score.

### (Usage Pattern)

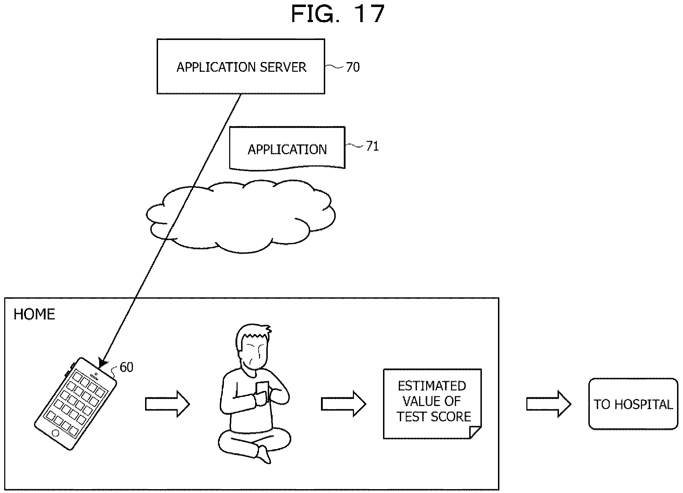

The estimation process described in the first embodiment may also be provided to each individual as an application. FIG. 17 is a diagram for explaining an exemplary usage pattern of a test score estimation application. As illustrated in FIG. 17, an application server 70 includes a first machine learning model 23 and a second machine learning model 24 trained by a preprocessing unit 40, and retains an estimation application (which will be referred to as an application hereinafter) 71 that executes processing similar to that of an operation processing unit 50.

In such a situation, a user purchases the application 71 at any place such as home, downloads the application 71 from the application server 70, and installs it on his/her own smartphone 60 or the like. Then, the user performs processing similar to that of the operation processing unit 50 described in the first embodiment using his/her own smartphone 60, and obtains a test score.

As a result, when the user goes to a hospital for a medical examination with the estimation result of the test score by the application, the hospital side is enabled to perform the medical examination in a state where the simple detection result is obtained, which may be useful for early determination of a disease name and symptom and an early start of treatment.

### (Numerical Values, etc.)

The exemplary numerical values, the training data, the explanatory variables, the objective variables, the number of devices, and the like used in the embodiment described above are merely examples, and may be optionally changed. In addition, the process flows described in the individual flowcharts may be appropriately modified unless otherwise contradicted.

### (System)

Pieces of information including the processing procedure, control procedure, specific names, various types of data, and parameters described above or illustrated in the drawings may be altered in any way unless otherwise noted.

Furthermore, each component of each device illustrated in the drawings is functionally conceptual, and is not necessarily physically configured as illustrated in the drawings. In other words, specific forms of distribution and integration of individual devices are not limited to those illustrated in the drawings. That is, all or a part thereof may be configured by being functionally or physically distributed or integrated in any units depending on various loads, usage conditions, or the like. For example, the preprocessing unit 40 and the operation processing unit 50 may be implemented by separate devices.

Moreover, all or any part of individual processing functions performed in each device may be implemented by a central processing unit (CPU) and a program analyzed and executed by the CPU, or may be implemented as hardware by wired logic.

### (Hardware)

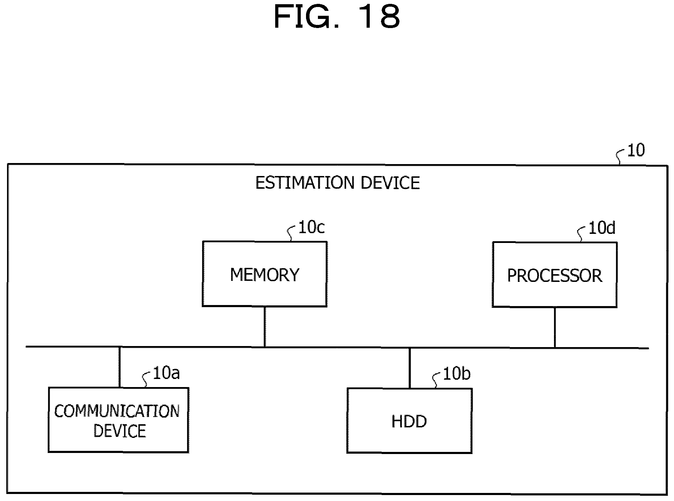

FIG. 18 is a diagram for explaining an exemplary hardware configuration. As illustrated in FIG. 18, the estimation device 10 includes a communication device 10a, a hard disk drive (HDD) 10b, a memory 10c, and a processor 10d. In addition, the respective units illustrated in FIG. 18 are mutually coupled by a bus or the like. Note that a display, a touch panel, and the like may be included in addition thereto.

The communication device 10a is a network interface card or the like, and communicates with another device. The HDD 10b stores programs and DBs for operating the functions illustrated in FIG. **2****.**

The processor 10d reads a program that executes processing similar to that of each processing unit illustrated in FIG. 2 from the HDD 10b or the like, and loads it into the memory 10c, thereby operating a process for executing each function described with reference to FIG. 2 and the like. For example, this process executes a function similar to that of each processing unit included in the estimation device 10. Specifically, the processor 10d reads, from the HDD 10b or the like, a program having functions similar to those of the preprocessing unit 40, the operation processing unit 50, and the like. Then, the processor 10d executes a process for performing processing similar to that of the preprocessing unit 40, the operation processing unit 50, and the like.

In this manner, the estimation device 10 operates as an information processing apparatus that executes an estimation method by reading and executing a program. Furthermore, the estimation device 10 may also implement functions similar to those of the embodiment described above by reading the program described above from a recording medium using a medium reading device and executing the read program described above. Note that the program referred to in other embodiments is not limited to being executed by the estimation device 10. For example, the embodiment described above may be similarly applied also to a case where another computer or server executes the program or a case where these cooperatively execute the program.

This program may be distributed via a network such as the Internet. In addition, this program may be recorded in a computer-readable recording medium such as a hard disk, a flexible disk (FD), a compact disc read only memory (CD-ROM), a magneto-optical disk (MO), or a digital versatile disc (DVD), and may be executed by being read from the recording medium by a computer.

## Claims

1. An estimation program for causing a computer to execute a process comprising:
obtaining video data that includes a face of a patient who performs a specific task;
detecting occurrence intensity of each of individual action units included in the face of the patient by inputting the obtained video data to a first machine learning model; and
estimating a test score of a test tool that executes a test related to dementia by inputting a temporal change in each of the detected occurrence intensity of the plurality of action units to a second machine learning model.

2. The estimation program according to claim **1,** the program causing the computer to execute the process further comprising:
training the test score of the test tool of the patient using the temporal change in the occurrence intensity of each of the plurality of action units as a feature to generate the second machine learning model.

3. The estimation program according to claim **1,** the program causing the computer to execute the process further comprising:
training the test score of the test tool of the patient using, as a feature, the temporal change in the occurrence intensity of each of the plurality of action units and a temporal change in face orientation of the patient to generate the second machine learning model.

4. The estimation program according to claim **1,** wherein the specific task includes an application or an interactive application that tests a cognitive function by loading the cognitive function.

5. The estimation program according to claim **1,** wherein the test score of the test tool includes a test result obtained by performing a mini mental state examination (MMSE), a Hasegawa's dementia scale-revised (HDS-R), or a Montreal cognitive assessment (MoCA), or any combination thereof.

6. An estimation method implemented by a computer, the estimation method comprising:
obtaining video data that includes a face of a patient who performs a specific task;
detecting occurrence intensity of each of individual action units included in the face of the patient by inputting the obtained video data to a first machine learning model; and
estimating a test score of a test tool that executes a test related to dementia by inputting a temporal change in each of the detected occurrence intensity of the plurality of action units to a second machine learning model.

7. An estimation device comprising:
a processor configured to:
obtain video data that includes a face of a patient who performs a specific task;
detect occurrence intensity of each of individual action units included in the face of the patient by inputting the obtained video data to a first machine learning model; and
estimate a test score of a test tool that executes a test related to dementia by inputting a temporal change in each of the detected occurrence intensity of the plurality of action units to a second machine learning model.
